(19) Europäisches Patentamt / European Patent Office / Office européen des brevets

(11) **EP 3 520 792 A1**

(12) **EUROPEAN PATENT APPLICATION**
published in accordance with Art. 153(4) EPC

(43) Date of publication:
**07.08.2019 Bulletin 2019/32**

(51) Int Cl.:
**A61K 31/445** $^{(2006.01)}$      **A61K 31/13** $^{(2006.01)}$

(21) Application number: **17856827.5**

(22) Date of filing: **29.09.2017**

(86) International application number:
**PCT/KR2017/010953**

(87) International publication number:
**WO 2018/062941 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.09.2016 KR 20160126230**
           **27.09.2017 KR 20170125220**

(71) Applicant: **Bio Pharmartis Co., Ltd.**
**Seoul 08506 (KR)**

(72) Inventors:
• **KOO, Hyoung Mo**
 **Suwon-si**
 **Gyeonggi-do 16435 (KR)**
• **KO, Chan Young**
 **Gimpo-si**
 **Gyeonggi-do 10120 (KR)**
• **LEE, Mase**
 **Goyang-si**
 **Gyeonggi-do 10229 (KR)**

(74) Representative: **Mewburn Ellis LLP**
**City Tower**
**40 Basinghall Street**
**London EC2V 5DE (GB)**

(54) **PHARMACEUTICAL COMPOSITION FOR PREVENTING OR TREATING DEMENTIA AND COGNITIVE DYSFUNCTION, CONTAINING DONEPEZIL OR PHARMACEUTICALLY ACCEPTABLE SALT THEREOF AND MEMANTINE OR PHARMACEUTICALLY ACCEPTABLE SALT THEREOF, AND PREPARATION METHOD THEREFOR**

(57) An immediate-release type pharmaceutical composition for preventing or treating dementia and cognitive dysfunction, including donepezil or a pharmaceutically acceptable salt thereof and memantine or a pharmaceutically acceptable salt thereof, and a method of preparing the pharmaceutical composition are disclosed. The method includes: granulating donepezil or a pharmaceutically acceptable salt thereof by a wet process; and adding memantine or a pharmaceutically acceptable salt thereof to the granulated donepezil or a pharmaceutically acceptable salt thereof and directly compressing and homogenizing the resulting mixture, thereby addressing problems such as reduced efficacy due to a decrease in elution of a donepezil active ingredient upon co-administration of Aricept® (donepezil hydrochloride) and Ebixa® (memantine hydrochloride), which are commercially available, and content uniformity is secured by selecting an excipient having a particle size similar to that of particles of the active ingredients. In addition, the pharmaceutical composition of the present invention is very stable without related substances.

FIG. 1A

Elution rate of donepezil hydrochloride (pH 1.2)

Control 1
Control 1 + Control 2
Example 1

EP 3 520 792 A1

**Description**

**TECHNICAL FIELD**

The present invention relates to an immediate-release type pharmaceutical composition for preventing or treating dementia and cognitive dysfunction, which includes, as active ingredients, donepezil or a pharmaceutically acceptable salt thereof and memantine or a pharmaceutically acceptable salt thereof, and a method of preparing the pharmaceutical composition.

**BACKGROUND ART**

With the development of the medical industry and rapid economic growth, quality of life has improved, and various diseases and the elderly population are also increasing, and accordingly, economic burden is increasing.

Among various diseases, brain function-related diseases have recently become a major issue, and due to causes including stress that is not controllable or avoidable, functional disorders such as fatigue, depression, insomnia, concentration/memory impairment, or oxidative brain damage occur. Also, the prevalence of neurodegenerative disorders, such as Alzheimer's dementia, Parkinson's disease, Huntington's disease and Lewy bodies, and consequent irreversible dementia, is gradually increasing.

More than 50% of senile dementia is Alzheimer's type dementia (AD).

Therapeutic agents for dementia or cognitive dysfunction were introduced into clinical practice in the 1990s, a series of acetylcholinesterase inhibitors (AChEi) such as donepezil, galantamine, rivastigmine, and the like were developed as therapeutic agents for Alzheimer's dementia, and memantine, which was developed as a therapeutic agent for Parkinson's disease and is an N-methyl-D-aspartate (NMDA) receptor antagonist, was approved as a therapeutic agent for Alzheimer's dementia.

Among acetylcholinesterase inhibitors, donepezil is administered starting at a dose of 5 mg once a day, administered at an increased dose of 10 mg after 4 to 6 weeks, and administered in the form of a solid preparation for oral administration, such as film-coated tablets, capsules, and granules.

Memantine was developed as a therapeutic agent for moderate to severe Alzheimer's disease and is administered in the form of a film-coated agent or a liquid preparation, and this drug is administered at a dose of 5 mg daily and then is administered at a maximum dose of 20 mg/day, i.e., 10 mg each for morning and evening with an increment of 5 mg every week, but many clinical researchers currently administer the drug once a day due to a long half-life of 60 to 80 hours and relatively high side effects.

According to a previous study (Tariot, 2004), in a case in which donepezil and memantine were co-administered to patients with moderate to severe Alzheimer's dementia, as a result of follow-up examination for 24 weeks, it was reported that this case exhibited both excellent cognitive function and excellent behavioral scores, as compared to a group treated only with donepezil (Pierre N. Tariot et al., [memantine Treatment in Patients with Moderate to Severe Alzheimer Disease Already Receiving Donepezil a Randomized Controlled trial], JAMA, Vol.291, No.3, p.317-324).

In addition, elderly patients, most of the patients with dementia, suffer from difficulty in swallowing, and their cognitive abilities are reduced, resulting in poor compliance with their own medication and low fidelity to medicine. Actually, there are research results showing that patients who take one pill exhibit an about 20% higher fidelity than patients who take two pills. As such, when combined drugs for treating dementia are developed, drug dose and drug fidelity may be increased.

Conventionally, Korean Patent Registration No. 1213345 discloses a technology for a combined preparation of donepezil and memantine and a technology for sustained release of one of the two drugs. However, in the case of the sustained-release type combined preparation, which is a preparation designed based on an *in vitro* test such as in an elution machine, it is not easy to produce products with a constant sustained release rate and it is also not easy to accurately predict the sustained release time due to a difference in gastrointestinal motility between individuals when people directly take the combined preparation. As such, two drugs with long half-lives, which are generally co-administered and expected to have no interaction, may be used as an immediate-release type preparation exhibiting efficacy within a short period of time.

Therefore, there is a need for an immediate-release type pharmaceutical composition that not only enhances a therapeutic effect by synergistic action between drugs, but also exhibits an elution rate similar to commercially available donepezil and commercially available memantine by using donepezil or a pharmaceutically acceptable salt thereof and memantine or a pharmaceutically acceptable salt thereof.

**DISCLOSURE OF INVENTION**

Therefore, the present invention has been made in view of the above problems, and it is an object of the present invention to provide an immediate-release type pharmaceutical composition for preventing or treating dementia and cognitive dysfunction, which includes, as active ingredients, donepezil or a pharmaceutically acceptable salt thereof and memantine or a pharmaceutically acceptable salt thereof.

It is another object of the present invention to provide a method of preparing an immediate-release type pharmaceutical composition for preventing or treating dementia and cognitive dysfunction, which includes, as active ingredients, donepezil or a pharmaceutically acceptable salt thereof and memantine or a pharmaceutically acceptable salt thereof.

In accordance with the present invention, the above and other objects can be accomplished by the provision of an immediate-release type pharmaceutical composition for preventing or treating dementia and cognitive dysfunction, which comprises memantine or a pharmaceutically acceptable salt thereof; and donepezil or a pharmaceutically acceptable salt thereof which is granulated by a wet process and is directly compressed in a form of tablets.

The granulated donepezil or a pharmaceutically acceptable salt thereof may be included in an amount of 1 wt% to 20 wt%.

The granulated donepezil or a pharmaceutically acceptable salt thereof and the memantine or a pharmaceutically acceptable salt thereof may be mixed in a weight ratio of 1:0.8 to 5.

The granulated donepezil or a pharmaceutically acceptable salt thereof may have a particle size distribution d(0.5) of 120 $\mu$m to 200 $\mu$m.

The granulated donepezil or a pharmaceutically acceptable salt thereof may include 15 wt% to 25 wt% of particles having a size of 20 mesh or more to less than 50 mesh, 25 wt% to 35 wt% of particles having a size of 50 mesh or more to less than 80 mesh, 20 wt% to 30 wt% of particles having a size of 80 mesh or more to less than 200 mesh, and 15 wt% to 25 wt% of particles having a size of at least 200 mesh, and the amount of the particles having a size of 20 mesh or more to less than 50 mesh may be smaller than that of the particles having a size of 200 mesh or more.

Particles having a size of 20 mesh of the granulated donepezil or a pharmaceutically acceptable salt thereof may be included in an amount of 5 wt% to 15 wt%.

The pharmaceutical composition may further include an excipient.

The excipient may be a mixture of lactose and microcrystalline cellulose in a weight ratio of 3 to 20:1.

The excipient may consist of particles having a size of 80 mesh or more included in an amount of 50 wt% or more.

In accordance with an aspect of the present invention, the above and other objects can be accomplished by the provision of a method of preparing an immediate-release type pharmaceutical composition for preventing or treating dementia and cognitive dysfunction, including granulating donepezil or a pharmaceutically acceptable salt thereof by a wet process, and adding memantine or a pharmaceutically acceptable salt thereof to the granulated donepezil or a pharmaceutically acceptable salt thereof and directly compressing and homogenizing the resulting mixture.

[Description of Drawings]

The above and other objects, features and other advantages of the present invention will be more clearly understood from the following detailed description taken in conjunction with the accompanying drawings, in which:

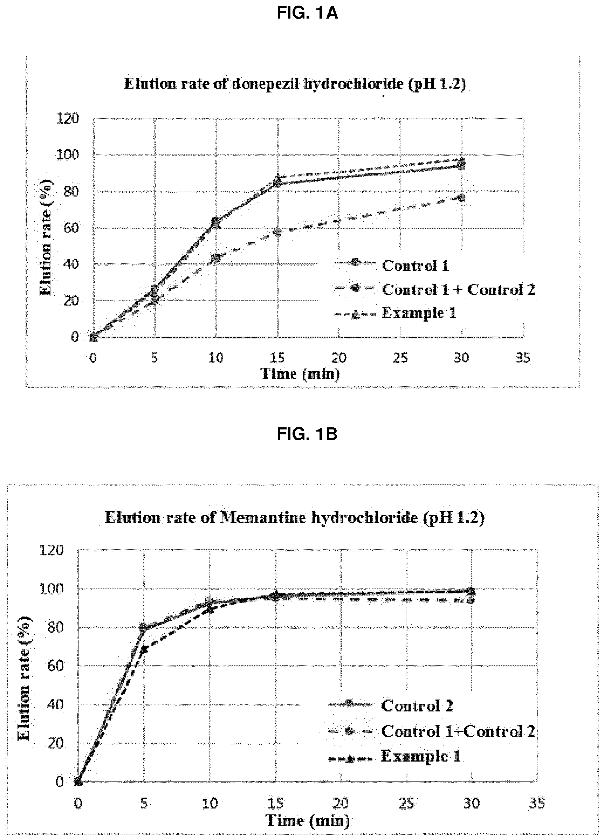

FIG. 1A is a graph showing measurement results of the elution rates of donepezil hydrochloride of a commercially available product of Control 1, mixed tablets of Controls 1 and 2, and combined tablets prepared according to Example 1 over time, and FIG. 1B is a graph showing measurement results of the elution rates of memantine hydrochloride of a commercially available product of Control 2, the mixed tablets of Controls 1 and 2, and the combined tablets of Example 1 over time;

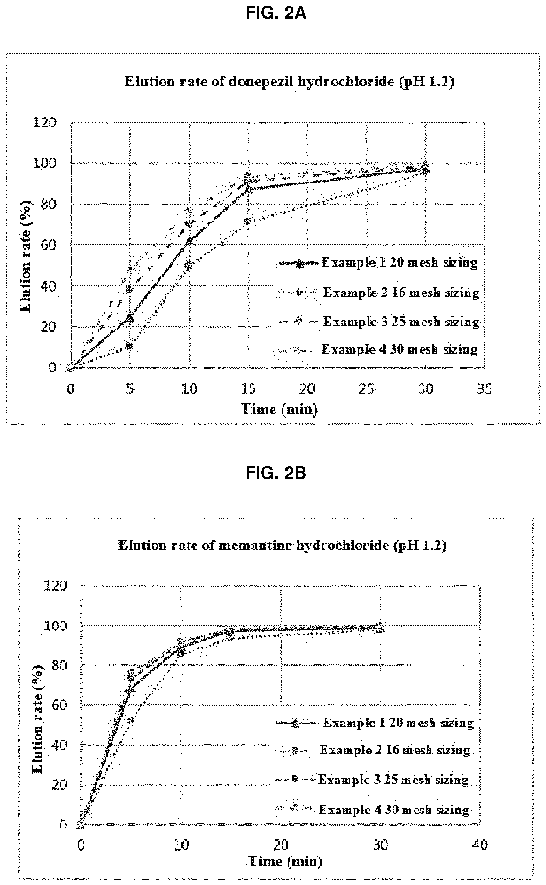

FIG. 2A is a graph showing measurement results of the elution rate of donepezil hydrochloride of combined tablets prepared according to Examples 1 to 4 over time, and FIG. 2B is a graph showing measurement results of the elution rate of memantine hydrochloride of the combined tablets of Examples 1 to 4 over time;

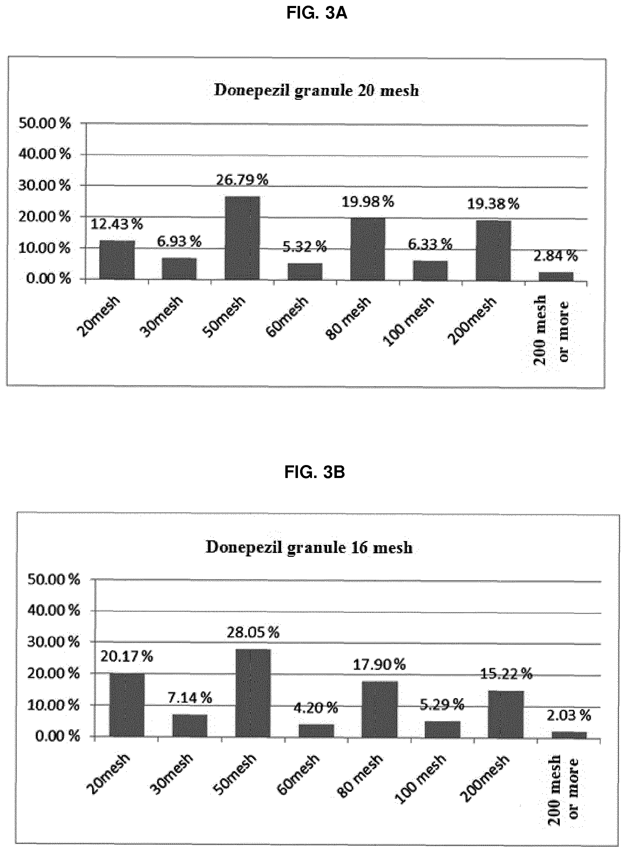

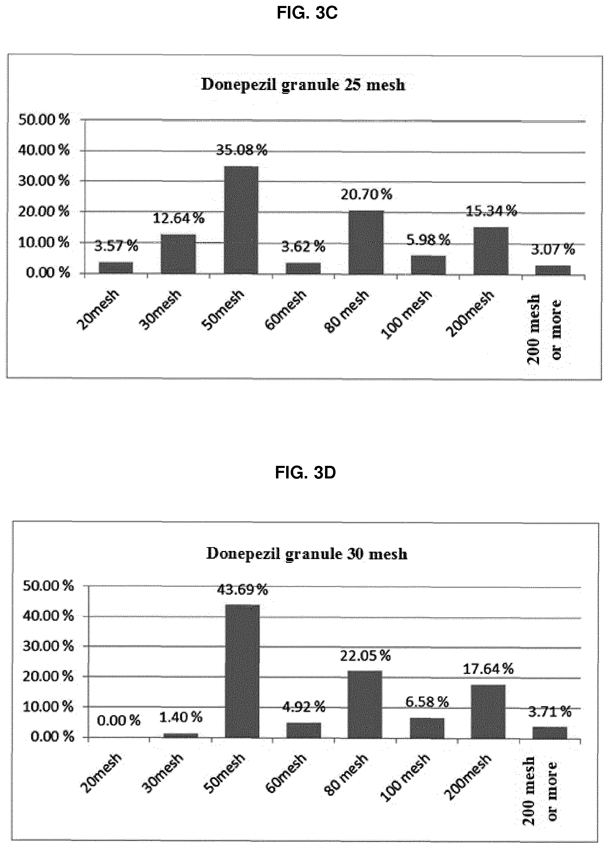

FIG. 3A is a graph showing the particle size of donepezil granules of Example 1, FIG. 3B is a graph showing the particle size of donepezil granules of Example 2, FIG. 3C is a graph showing the particle size of donepezil granules of Example 3, and FIG. 3D is a graph showing the particle size of donepezil granules of Example 4;

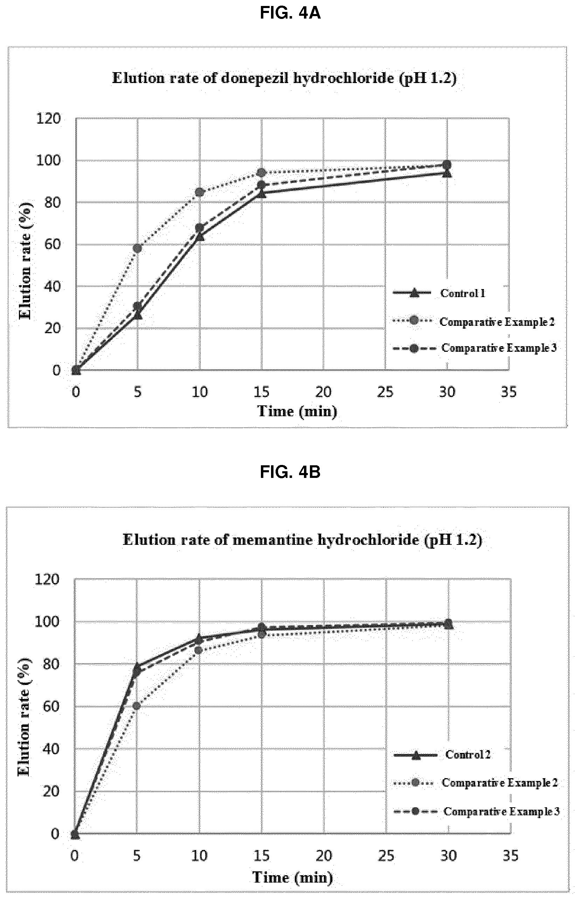

FIG. 4A is a graph showing measurement results of the elution rate of donepezil hydrochloride of Control 1 and tablets prepared according to Comparative Examples 2 and 3 over time, and FIG. 4B is a graph showing measurement results of the elution rate of memantine hydrochloride of Control 1 and the tablets of Comparative Examples 2 and 3 over time;

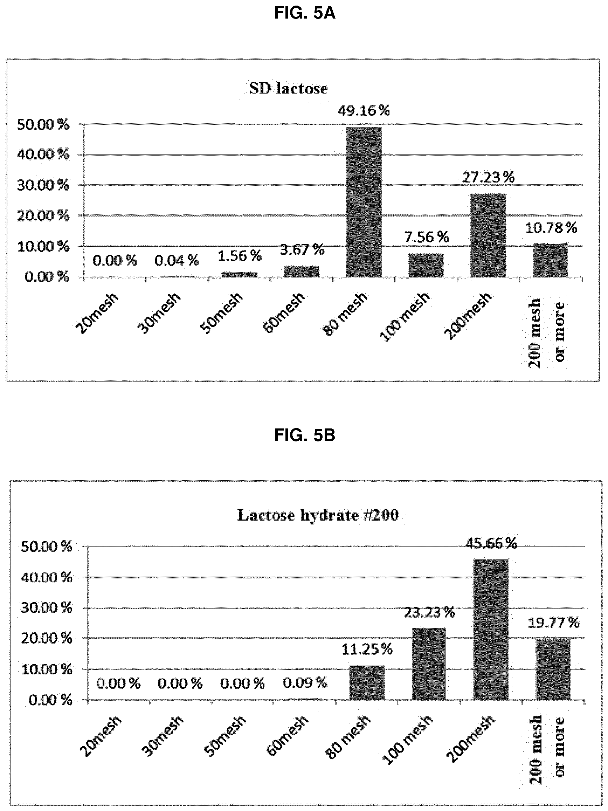

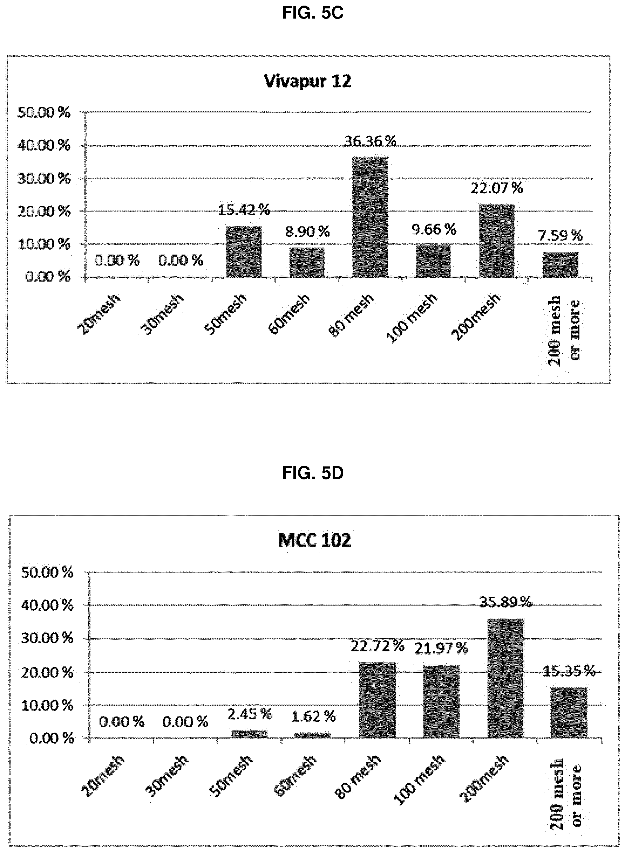

FIG. 5A is a graph showing the particle size of SD lactose, FIG. 5B is a graph showing the particle size of lactose hydrate, FIG. 5C is a graph showing the particle size of microcrystalline cellulose, Vivapur 12, and FIG. 5D is a graph showing microcrystalline cellulose, MCC 102; and

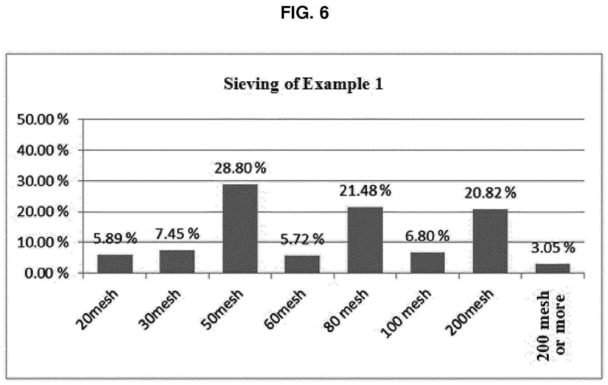

FIG. 6 is a graph showing the sieving particle size of the combined tablets of Example 1 using lactose and microcrystalline cellulose having the above-described particle sizes.

**BEST MODE FOR CARRYING OUT THE INVENTION**

Reference will now be made in detail to the preferred embodiments of the present invention, examples of which are illustrated in the accompanying drawings. Wherever possible, the same reference numbers will be used throughout the drawings to refer to the same or like parts.

The present disclosure relates to an immediate-release type pharmaceutical composition for preventing or treating dementia and cognitive dysfunction, which includes donepezil or a pharmaceutically acceptable salt thereof and memantine or a pharmaceutically acceptable salt thereof, and a method of preparing the pharmaceutical composition.

Donepezil hydrochloride is formulated and is currently marketed under the trade name 'Aricept®' (containing 5 mg or 10 mg of donepezil hydrochloride), and memantine hydrochloride is formulated and is currently marketed under the trade name 'Ebixa®' (containing 10 mg of memantine hydrochloride). As a result of an *in vitro* (elution test) test of commercially available Aricept® and Ebixa®, it was found that, while the same results were obtained in the case of Ebixa® for a single elution rate and a combined elution rate (Aricept® + Ebixa®), the single elution rate and the combined elution rate (Aricept® + Ebixa®) were significantly reduced in the case of Aricept®. This is because, due to physical interactions between Ebixa® and Aricept® at the time of elution, the combined elution rate of Aricept was 20% or more lower than the single elution rate thereof in 15 minutes. Such an elution deterioration phenomenon significantly affects the drug absorption rate and bioavailability of donepezil so that desired efficacy cannot be exhibited.

An initial 5-minute elution rate of donepezil or pharmaceutically acceptable salts thereof may be suppressed to 35% or less due to the characteristics of the active ingredient, such as digestive system side effects such as nausea, vomiting, and diarrhea, and mental nervous system side effects such as muscle spasms, fatigue, and dizziness.

In tablet form, in which donepezil or a pharmaceutically acceptable salt thereof and memantine or a pharmaceutically acceptable salt thereof are homogenously mixed without separation as in double-layered tablets, multilayered tablets, or the like, it is difficult to realize the elution rates of donepezil and memantine ingredients, which belong to the biopharmaceutics classification system (BCS) class I having high solubility, to 35% or less and 60% or more, respectively, over the initial 5 minutes like the elution rate of commercially available preparations, by using a general method, not by a special method.

Thus, in the present invention, to suppress the initial elution rate of donepezil, wet granulation was carried out, and to improve the combined preparation (Aricept® + Ebixa®) in which the elution rate of the donepezil is 20% or more lower in 15 minutes, the particle size of donepezil or a pharmaceutically acceptable salt thereof was set.

Conventionally, Korean Patent Publication No. 2009-0016611 discloses that, when memantine is produced by a direct compression method, content uniformity is lowered, and adhesiveness and capping problems occur, and thus it is necessary to granulate memantine by a wet process. In the present invention, however, memantine was produced using a direct compression method capable of reducing costs and time while maintaining advantages (tabletability, mixing uniformity, and capping) obtained by performing a wet process.

In addition, when a combined preparation is prepared by mixing donepezil or a pharmaceutically acceptable salt thereof and memantine or a pharmaceutically acceptable salt thereof and directly compressing the resulting mixture, costs and time may be reduced by simplifying manufacturing processes, but it may be difficult to reduce the initial 5-minute elution rate of donepezil. Moreover, when a combined preparation is prepared by mixing donepezil or a pharmaceutically acceptable salt thereof and memantine or a pharmaceutically acceptable salt thereof and performing wet granulation on the resulting mixture or by respectively performing wet granulation on the same, related substances of memantine occur due to interactions with donepezil.

For example, as a result of confirming related substances through a stability test of combined preparations prepared by (a) wet granulating donepezil or a pharmaceutically acceptable salt thereof and wet granulating memantine or a pharmaceutically acceptable salt thereof; (b) wet granulating donepezil or a pharmaceutically acceptable salt thereof and directly compressing memantine or a pharmaceutically acceptable salt thereof; (c) mixing donepezil or a pharmaceutically acceptable salt thereof and memantine or a pharmaceutically acceptable salt thereof and directly compressing the resulting mixture; (d) directly compressing donepezil or a pharmaceutically acceptable salt thereof and wet granulating memantine or a pharmaceutically acceptable salt thereof; and (e) mixing donepezil or a pharmaceutically acceptable salt thereof and memantine or a pharmaceutically acceptable salt thereof and wet granulating the resulting mixture, greater amounts of related substances were generated in the preparation processes (a), (d), and (e) than in the preparation processes (b) and (c).

Therefore, the inventors of the present invention confirmed that a combined preparation including donepezil or a pharmaceutically acceptable salt thereof and memantine or a pharmaceutically acceptable salt thereof, which have different action mechanisms, had an improved therapeutic effect due to synergistic action between the two drugs, had an elution rate similar to that of commercially available donepezil and commercially available memantine, secured tabletability, and barely cause the generation of degradation products (related substances), and was very stably stored, and thus completed the present invention.

Hereinafter, the present invention will be described in detail.

An immediate release type pharmaceutical composition for preventing or treating dementia and cognitive dysfunction of the present invention includes memantine or a pharmaceutically acceptable salt thereof and wet-granulated donepezil or a pharmaceutically acceptable salt thereof and is directly compressed (hereinafter referred to as a combined preparation), and may be in the form of tablets among various forms such as tablets, capsules, injections, and the like.

In particular, a method of preparing an immediate-release type pharmaceutical composition for preventing or treating dementia and cognitive dysfunction of the present invention includes: granulating donepezil or a pharmaceutically acceptable salt thereof by a wet process; and adding memantine or a pharmaceutically acceptable salt thereof to the granulated donepezil or a pharmaceutically acceptable salt thereof and directly compressing and homogenizing the resulting mixture.

When two drugs having different action mechanisms are mixed, the mixture is unstable in the presence of moisture, and thus the number of related substances may be rapidly increased due to physical and chemical properties thereof. Thus, to overcome this, in the present invention, donepezil or a pharmaceutically acceptable salt thereof is first granulated by a wet process, and then memantine or a pharmaceutically acceptable salt thereof and an additive are added thereto and the resulting mixture is directly compressed.

First, in the present invention, donepezil or a pharmaceutically acceptable salt thereof is granulated by a wet process to adjust an initial elution rate thereof. That is, donepezil or a pharmaceutically acceptable salt thereof may be granulated by a wet process, and then mixed with memantine or a pharmaceutically acceptable salt thereof, followed by direct compression, thereby inhibiting the elution of the donepezil or a pharmaceutically acceptable salt thereof over the initial 5 minutes. Preferably, the initial 5-minute elution may be suppressed to 35% or less.

The donepezil is a compound having the chemical name of 1-benzyl-4-[(5,6-dimethoxy-1-indanon-2-yl)methyl]piperidine, and donepezil or a pharmaceutically acceptable salt thereof is known as a useful drug for the prevention of senile dementia, especially for the prevention and treatment of Alzheimer's dementia.

The pharmaceutically acceptable salt of donepezil is not particularly limited, but may be any salt that increases the solubility of donepezil, preferably an acid addition salt.

The pharmaceutically acceptable salts must have low human toxicity and must not adversely affect the biological activity and physicochemical properties of a parent compound. In addition, free acids that may be used in preparation of the pharmaceutically acceptable salts may be divided into inorganic acids and organic acids. As the inorganic acid, hydrochloric acid, sulfuric acid, nitric acid, phosphoric acid, perchloric acid, bromic acid, or the like may be used. As the organic acid, acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, benzoic acid, pamoic acid, aspartic acid, glutamic acid, or the like may be used. An organic base that may be used in preparation of an organic base addition salt includes tris(hydroxymethyl)methylamine, dicyclohexylamine, and the like. Amino acids that may be used in the preparation of amino acid addition bases include natural amino acids such as alanine, glycine, and the like. In addition, the donepezil according to the present invention may also include any hydrate or solvate of donepezil, in addition to the pharmaceutically acceptable salt thereof. The hydrate or solvate of donepezil may be prepared by dissolving donepezil in a solvent mixable with water, such as methanol, ethanol, acetone, or 1,4-dioxane, adding a free acid or a free base thereto, and crystallizing or recrystallizing the resulting solution. In this case, a solvate (especially a hydrate) may be formed. Thus, the compound of the present invention may also include a stoichiometric solvate including a hydrate, in addition to various amounts of water-containing compounds that can be prepared using a method such as lyophilization.

The pharmaceutically acceptable salt of donepezil is preferably a hydrochloride.

The amount of the donepezil or a pharmaceutically acceptable salt thereof in the pharmaceutical composition of the present invention ranges from 1 wt% to 20 wt%, preferably 2 wt% to 10 wt%. When the amount of the donepezil or a pharmaceutically acceptable salt thereof is less than the above lower limit, the mass of combined tablets is 521.5 mg or more, and thus the convenience of dosing may deteriorate. When the amount of the donepezil or a pharmaceutically acceptable salt thereof is greater than the above-described upper limit, the mass of combined tablets is less than 104.3 mg and it may not be easy to prepare a combined preparation in combination with memantine or a pharmaceutically acceptable salt thereof.

In addition, the donepezil or a pharmaceutically acceptable salt thereof is granulated by a wet process due to the characteristics of the active ingredient having a high frequency of digestive system side effects such as nausea, vomiting, and diarrhea and mental nervous system side effects such as muscle spasms, fatigue, and dizziness, thereby suppressing the initial 5-minute elution to 35% or less so that the initial 5-minute elution is set at the same level as that of a commercially available donepezil drug (Aricept®).

In addition, when the donepezil or a pharmaceutically acceptable salt thereof is subjected to a direct compression process instead of the wet process, it is impossible to secure a suitable initial elution rate (a 5-minute elution rate of 35% or less), and when the donepezil or a pharmaceutically acceptable salt thereof is mixed with memantine or a pharmaceutically acceptable salt thereof in a subsequent process, a composition exhibiting digestive system side effects such as nausea, vomiting, and diarrhea, and metal nervous system side effects such as muscle spasms, fatigue, and dizziness may be prepared.

The wet granulation method includes granulating, in a liquid, donepezil or a pharmaceutically acceptable salt thereof and a first pharmaceutically acceptable additive, preferably one or more selected from an excipient, a binder, a diluent, and a disintegrating agent to form granules and drying and oscillating the granules. The scope of the present invention is not limited to the use of the first additive, and in addition to the above-described additives, commonly used additives selected by those of ordinary skill in the art within the scope that does not adversely affect the effects of the present invention may be used.

The excipient may include, but is not limited to, at least one selected from the group consisting of lactose, microcrystalline cellulose, mannitol, starch, and corn starch; the binder may include, but is not limited to, at least one selected from the group consisting of polyvinylpyrrolidone, polyvinyl alcohol, hydroxypropyl methylcellulose, hydroxy propylcellulose, ethylcellulose, and methylcellulose; the diluent may include, but is not limited to, calcium phosphate (dibasic and/or tribasic), calcium sulfate, powdered cellulose, dextrates, dextrin, fructose, kaolin, lactitol, lactose, maltose, mannitol, microcrystalline cellulose, sorbitol, starch, and sucrose; and the disintegrating agent may include, but is not limited to, at least one selected from croscarmellose sodium, crospovidone, microcrystalline cellulose, polyacrolein potassium, sodium starch glycolate, low substituted hydroxypropyl cellulose, and starch.

Next, in the present invention, memantine or a pharmaceutically acceptable salt thereof and a second additive are added to the granulated donepezil or a pharmaceutically acceptable salt thereof and mixed therewith and the resulting mixture is directly compressed and homogenized, so that the active ingredients are uniformly distributed.

Memantine is a compound having the chemical name of 3,5-dimethyladamantan-1-amine and has been reported as an orally active NMDA receptor antagonist, and memantine or a pharmaceutically acceptable salt thereof is known as a useful drug for the prevention and treatment of Alzheimer's disease.

The pharmaceutically acceptable salt of memantine is not particularly limited, but may be any salt that increases the solubility of donepezil, preferably an acid addition salt, and more preferably a hydrochloride.

The donepezil or a pharmaceutically acceptable salt thereof and the memantine or a pharmaceutically acceptable salt thereof are mixed in a weight ratio of 1:0.8 to 5, preferably in a weight ratio of 1:1 to 3. When the amount of the memantine or a pharmaceutically acceptable salt thereof exceeds the above-described range with respect to the amount of the donepezil or a pharmaceutically acceptable salt thereof, digestive system side effects such as nausea, vomiting, and diarrhea and mental nervous system side effects such as muscle spasms, fatigue, and dizziness may occur.

In addition, a mixture of the memantine or a pharmaceutically acceptable salt thereof, an additive, and the granulated donepezil or a pharmaceutically acceptable salt thereof is homogenized by a direct compression process, and when a wet process or a direct compression process after a wet process is performed instead of a direct compression process, a large amount of a related substance occurs.

The homogenization method by a direct compression process is a method of directly compressing the above-described ingredients without physical change of a combined preparation, and compression by a rotary tablet press includes homogenizing powder by upper and lower punches, reducing the homogenized material to a granule size, and then compressing the resulting material.

The second additive may be one or more selected from an excipient, a binder, a lubricant, and a coating agent, which are pharmaceutically acceptable. The scope of the present invention is not limited to use of the second additive, and in addition to the above-described additives, commonly used additives selected by those of ordinary skill in the art within the scope that does not adversely affect the effects of the present invention may be used.

The types of the excipient and the binder are the same as those described in the first additive. The lubricant may include, but is not limited to, one or more selected from colloidal silicon dioxide, hydrous silicon dioxide, magnesium stearate, and sodium stearyl fumarate, and the coating agent may include, but is not limited to, hydroxypropyl methyl cellulose, polyvinylpyrrolidone, copovidone, Opadry® series, and Eudragit® series.

In the above process, to homogenously mix memantine present in a particulate form or a pharmaceutically acceptable salt thereof with donepezil having a relatively large particle size or a pharmaceutically acceptable salt thereof, it is required to perform trituration mixing by adding an excipient mixture of a particulate excipient and an excipient having a relatively large particle size.

The particulate excipient included in the excipient mixture has to have a particle size similar to that of memantine present in a particulate form or a pharmaceutically acceptable salt thereof, and the excipient having a relatively large particle size included in the excipient mixture has to have a particle size similar to that of donepezil having a relatively large particle size or a pharmaceutically acceptable salt thereof. When the particle size of each excipient included in the excipient mixture is not similar to that of memantine or a pharmaceutically acceptable salt thereof and donepezil or a pharmaceutically acceptable salt thereof, a combined preparation including memantine or a pharmaceutically acceptable salt thereof and donepezil or a pharmaceutically acceptable salt thereof may not satisfy an acceptable range in terms of content uniformity.

For example, the particle size distribution d(0.5) of the excipient with respect to the d(0.5) (donepezil d(0.5) μm) of donepezil or a pharmaceutically acceptable salt thereof which is granulated by a wet process, is donepezil d(0.5)

$\mu$m $\pm$ 55 to 60 $\mu$m, the particle size distribution d(0.9) of the excipient with respect to the d(0.9) (donepezil d(0.9) $\mu$m) is donepezil d(0.9) $\mu$m $\pm$ 380 to 400 $\mu$m, the particle size distribution d(0.5) of the excipient with respect to the d(0.5) (memantine d(0.5) $\mu$m) of memantine or a pharmaceutically acceptable salt thereof is memantine d(0.5) $\mu$m $\pm$ 55 to 60 $\mu$m, and the particle size distribution d(0.9) of the excipient with respect to the d(0.9) (memantine d(0.9) $\mu$m) is memantine d(0.9) $\mu$m $\pm$ 90 to 95 $\mu$m. Also, assuming that a difference between the d(0.5) values of memantine or a pharmaceutically acceptable salt thereof and donepezil or a pharmaceutically acceptable salt thereof is 100, a difference between the particle size distribution d(0.5) values of the excipients included in the excipient mixture ranges from 93 to 105, and assuming that a difference between the d(0.9) values of memantine or a pharmaceutically acceptable salt thereof and donepezil or a pharmaceutically acceptable salt thereof is 100, a difference between the particle size distribution d(0.9) values of the excipients included in the excipient mixture may range from 30 to 50. The d(0.5) and d(0.9) mean a size corresponding to 50% and 90% of the maximum value in the cumulative distribution measured by a particle size analyzer.

As powder becomes finer, specific surface area increases and adhesion cohesiveness between powders increases. For example, when an excipient having a considerably larger particle size than that of the active ingredient having a small particle size is used, the active ingredient having a small particle size is not sufficiently and uniformly mixed with the excipient and is partially agglomerated or concentrated, thus causing mixing non-uniformity, and the opposite case also causes the occurrence of the same phenomenon.

Thus, to adjust the initial 5-minute elution rate of donepezil or a pharmaceutically acceptable salt thereof to 35% or less, the wet-granulated particles may have a particle size distribution d(0.5) of 120 $\mu$m to 200 $\mu$m. When d(0.5) is less than the above-described lower limit, an elution rate of greater than 35% is obtained at the time of elution over the initial 5 minutes, and when d(0.5) is greater than the above-described upper limit, the elution rate is not greater than 35% at the time of elution over the initial 5 minutes, but 70% that is much lower than the elution rate (85%) of Aricept® as a control in 15 minutes.

Furthermore, the donepezil or a pharmaceutically acceptable salt thereof including 15 wt% to 25 wt% of particles having a size of 20 mesh or more to less than 50 mesh, 25 wt% to 35 wt% of particles having a size of 50 mesh or more to less than 80 mesh, 20 wt% to 30 wt% of particles having a size of 80 mesh or more to less than 200 mesh, and 15 wt% to 25 wt% of particles having a size of at least 200 mesh exhibits an elution rate of not greater than 35% at the time of elution over the initial 5 minutes. More preferably, a case in which the amount of particles having a size of 20 mesh to less than 50 mesh is less than that of particles having a size of 200 mesh or more exhibits an elution rate of not greater than 35% at the time of elution over the initial 5 minutes and an elution rate higher than that (85%) of Aricept® as a control in 15 minutes.

In addition, to achieve mixing uniformity after directly compressing the wet-granulated donepezil or a pharmaceutically acceptable salt thereof, and memantine or a pharmaceutically acceptable salt thereof which are added afterward, in one embodiment, content uniformity was secured by adjusting the ratio of lactose (spray-dried lactose) (d(0.1) 23.1 $\mu$m, d(0.5) 69.2 $\mu$m, d(0.9) 164.9 $\mu$m) and microcrystalline cellulose (Vivapur 12; d(0.1) 153.5 $\mu$m, d(0.5) 193.0 $\mu$m, d(0.9) 386.7 $\mu$m).

More preferably, when lactose and microcrystalline cellulose having a particle size of 80 mesh or more are included in an amount of 50 wt% or more, excellent content uniformity may be secured.

The weight ratio of the lactose to the microcrystalline cellulose is in a range of 3 to 20:1, preferably 5 to 15:1. When the amount of the lactose exceeds the above-described range with respect to the amount of the microcrystalline cellulose, it may be difficult to suppress the initial 5-minute elution rate of donepezil or a pharmaceutically acceptable salt thereof to 35% or less.

In the pharmaceutical composition of the present invention, under a condition of a pH 1.2 eluate, the initial 5-minute elution rate of donepezil or a pharmaceutically acceptable salt thereof is suppressed to 35% or less, and the initial 5-minute elution rate of memantine or a pharmaceutically acceptable salt thereof is 60% or more. In addition, in the pharmaceutical composition of the present invention, given a pH 1.2 eluate, donepezil or a pharmaceutically acceptable salt thereof is eluted in an amount of 75% or more, preferably 80% or more with respect to the total weight of the active ingredient in 15 minutes, and memantine or a pharmaceutically acceptable salt thereof is eluted in an amount of 85% or more, preferably 85% to 95% with respect to the total weight of the active ingredient in 15 minutes and in an amount of 90% or more with respect to the total weight of the active ingredient in 30 minutes.

A selected dosage level of the composition may vary depending on the activity of compounds used, administration routes, the severity of condition being treated, the condition of patients being treated, and previous medical history. However, it is within the knowledge of those skilled in the art to gradually increase the dose of the compound until the desired effect is achieved, starting with the dose of the compound at a level lower than that required to achieve the desired therapeutic effect, and a suitable dose may be determined by age, gender, body shape, and body weight. The composition may be further processed before being formulated into a pharmaceutically acceptable pharmaceutical preparation, and is preferably pulverized or ground into smaller particles. In addition, the composition may vary depending on the condition of patients being treated, but may be determined non-creatively.

Hereinafter, exemplary embodiments will be described to aid in understanding of the present invention, it will be obvious to those of ordinary skill in the art that the following examples are provided for illustrative purposes only and various changes and modifications can be made within the sprit and scope of the present invention, and such changes and modifications should be within the scope of the appended claims.

**Control 1: Commercial Preparation Aricept® 10 mg**

10 mg of Aricept® (available from Eisai Korea) containing 10 mg of donepezil hydrochloride was used.

**Control 2: Commercial Preparation Ebixa® 10 mg X 2**

20 mg (10 mg X 2) of Ebixa® (available from Lundbeck Korea Co., Ltd) containing 10 mg of memantine hydrochloride was used.

**Example 1: Combined Tablets**

41.72 g (10.4 mg) of donepezil hydrochloride monohydrate, 480 g (120 mg) of mannitol, and 20 g (5 mg) of hypromellose were mixed. Separately, 20 g (5 mg) of hydroxypropyl cellulose was added to 120 g of purified water to dissolve the mixture, and then the mixture was added to the donepezil hydrochloride mixture and mixed using a speed mixer. The mixture was then dried at 60 °C for 2 hours and filtered through a 20-mesh filter oscillator to be sized (d(0.1) 9.6 $\mu$m, d(0.5) 141.8 $\mu$m, d(0.9) 784.3 $\mu$m)

80 g of memantine hydrochloride (20 mg; d(0.1) 1.9 $\mu$m, d(0.5) 12.3 $\mu$m, d(0.9) 73.7 $\mu$m), 492 g (123 mg) of SD lactose (d(0.1) 23.2 $\mu$m, d(0.5) 69.2 $\mu$m, d(0.9) 164.9 $\mu$m), and 48 g (12 mg) of Vivapur 12 (d(0.1) 153.5 $\mu$m, d(0.5) 193.0 $\mu$m, d(0.9) 386.7 $\mu$m) were filtered through a 20-mesh filter and mixed, and then the filtered donepezil tablets and 16 g (4 mg) of magnesium stearate were further added thereto and mixed. Subsequently, the mixture of donepezil and memantine was compressed using a rotary tablet press to produce tablets, followed by film coating with 40 g (10 mg) of Opadry® as a coating agent.

The case of Example 1 was prepared to satisfy 10 mg of donepezil hydrochloride and 20 mg of memantine hydrochloride.

**Example 2: Combined Tablets_16 mesh Sizing**

Preparation was performed in the same manner as in Example 1, except that the mixture was filtered using a 16 mesh oscillator and subjected to sizing, thereby obtaining a sized donepezil product.

**Example 3: Combined Tablets_25 mesh Sizing**

Preparation was performed in the same manner as in Example 1, except that the mixture was filtered using a 25 mesh oscillator and subjected to sizing, thereby obtaining a sized donepezil product.

**Example 4: Combined Tablets_30 mesh Sizing**

Preparation was performed in the same manner as in Example 1, except that the mixture was filtered using a 30 mesh oscillator and subjected to sizing, thereby obtaining a sized donepezil product.

**Comparative Example 1: Combined Tablets_Small Particle Size Distribution**

A filtered and sized donepezil product was prepared in the same manner as in Example 1.

80 g (20 mg) of memantine (d(0.1) 1.9 $\mu$m, d(0.5) 12.3 $\mu$m, d(0.9) 73.7 $\mu$m), 492 g (123 mg) of hydrous lactose (d(0.1) 4.6 $\mu$m, d(0.5) 15.8 $\mu$m, d(0.9) 66.7 $\mu$m), and 48 g (12 mg) of microcrystalline cellulose (d(0.1) 20.1 $\mu$m, d(0.5) 61.2 $\mu$m, d(0.9) 157.2 $\mu$m) were filtered through a 20-mesh filter and mixed, and then the filtered and sized donepezil product and 16 g (4 mg) of magnesium stearate were further added thereto and mixed. Subsequently, the mixture of donepezil and memantine was compressed using a rotary tablet press to produce tablets, followed by film coating with 40 g (10 mg) of Opadry® as a coating agent.

The case of Example 2 was also prepared so as to satisfy 10 mg of donepezil hydrochloride and 20 mg of memantine hydrochloride.

**Comparative Example 2: Direct Compression after Wet Process of Mixture of Donepezil Hydrochloride + Memantine Hydrochloride**

The same materials and amounts as those used in Example 1 were used.

41.72 g of donepezil hydrochloride monohydrate, 480 g of mannitol, 20 g of hypromellose, and 80 g of memantine hydrochloride were mixed. Separately, 20 g of hydroxypropylcellulose was added to 150 g of purified water to dissolve the mixture, and then the mixture was added to the donepezil hydrochloride mixture and mixed using a speed mixer. The mixture was then dried at 60 °C for 2 hours and sized using an oscillator. 492 g of SD lactose and 48 g of Vivapur 12 were filtered through a 20-mesh filter, and the filtrate was added to the mixture of donepezil and memantine and mixed, and then 16 g of magnesium stearate was further added thereto and mixed. Thereafter, the mixture of donepezil and memantine was compressed using a rotary tablet press to formulate into tablets, followed by film coating with 40 g of Opadry® as a coating agent.

**Comparative Example 3: Direct Compression after Donepezil hydrochloride Wet Process + Memantine Hydrochloride Wet Process**

The same materials and amounts as those used in Example 1 were used.

41.72 g of donepezil hydrochloride monohydrate, 480 g of mannitol, and 20 g of hypromellose were mixed. Separately, 20 g of hydroxypropylcellulose was added to 120 g of purified water to dissolve the mixture, and then the mixture was added to the donepezil hydrochloride mixture and mixed using a speed mixer.

80 g of memantine hydrochloride and 492 g of SD lactose were mixed, and then 180 g of purified water was added thereto and mixed using a speed mixer.

The donepezil hydrochloride mixture and memantine hydrochloride mixture were dried at 60 °C for 2 hours, and then sized by an oscillator and filtered through a 20-mesh filter. Then, 48 g of Vivapur 12 filtered through a 20-mesh filter was added thereto and mixed and 16 g of magnesium stearate was added thereto and mixed. Thereafter, the mixture of donepezil and memantine was compressed using a rotary tablet press to produce tablets, followed by film coating with 40 g of Opadry® as a coating agent.

**Comparative Example 4: Direct Compression after Simple Mixing of Donepezil hydrochloride and Memantine Hydrochloride**

41.72 g of donepezil hydrochloride monohydrate, 480 g of mannitol, 20 g of hypromellose, 80 g of memantine, 20 g of hydroxypropylcellulose, 492 g of SD lactose, and 48 g of Vivapur 12 were filtered with 20 mesh and mixed, and then 16 g of magnesium stearate was further added thereto and mixed. Thereafter, the mixture of donepezil and memantine was compressed using a rotary tablet press to produce tablets, followed by film coating with 40 g of Opadry® as a coating agent.

**<Experimental Examples>**

**Experimental Example 1: Elution Test**

An elution test was performed on Controls 1 and 2 and the combined tablets of Example 1 under the following conditions.

Control 1: Control 1 (Aricept® 10 mg) single elution
Control 2: Control 2 (Ebixa® 10 mg X 2 tablets) single elution
Controls 1 and 2: simple mixing of Control 1 (Aricept® 10 mg) and Control 2 (Ebixa® 10 mg X 2 tablets) and then combined elution

Example 1: Combined Tablets

[Table 1]

| <Elution conditions> | |
|---|---|
| Eluate | Solution 1 (pH 1.2) in Disintegration Test in General Test Methods of the Korean Pharmacopoeia |

(continued)

| <Elution conditions> | |
|---|---|
| Amount of eluate | 900 ml |
| Device | USP paddle method, 50 rpm |
| Temperature | 37.0 ± 0.5 °C |
| <Analysis conditions> | |
| Column | YMC ODS-AQ (4.6 mm X 150 mm, 5 um) or column similar thereto |
| Mobile phase | Methanol: buffer=60:40(v/v)<br>Buffer: 10 ml of triethylamine was added to 1 L of purified water and the pH of the resulting solution was adjusted to 5.5 with glacial acetic acid |
| Flow rate | About 0.9 ml/min |
| Column temperature | 50 °C |
| Detector temperature | 50 °C |
| Dose | 50 μl |
| Detector | Refractive index detector |

FIG. 1A is a graph showing measurement results of the elution rates of donepezil hydrochloride of a commercially available product of Control 1, mixed tablets of Controls 1 and 2, and combined tablets prepared according to Example 1 over time, and FIG. 1B is a graph showing measurement results of the elution rates of memantine hydrochloride of a commercially available product of Control 2, the mixed tablets of Controls 1 and 2, and the combined tablets of Example 1 over time.

For single elution, the elution rate of Control 1 is less than 35% for the initial 5 minutes and 80% or more during 15 minutes. However, upon co-elution, the elution rate of a combination of Controls 1 and 2 was less than 35% for the initial 5 minutes, but less than 60% for 15 minutes, from which it was confirmed that elution rate reduction was exhibited due to physical interference of Control 2.

In the case of Example 1, upon co-elution of donepezil hydrochloride and memantine hydrochloride, which were homogenously mixed without separation, it was confirmed that the elution rate of donepezil was less than 35% for 5 minutes and 80% or more during 15 minutes, and the elution rate of memantine was 60% or more during 5 minutes and 90% or more during 15 minutes.

Thus, as illustrated in FIGS. 1A and 1B, the combined tablets of Example 1 of the present invention were prepared such that, similar to Control 1, which is a commercial product, the elution rate of donepezil hydrochloride was suppressed to less than 35% for the initial 5 minutes, and similar to Control 2, the elution rate of memantine hydrochloride was 60% or more for the initial 5 minutes. It was also confirmed that, in the combined tablets of Example 1, the donepezil hydrochloride was eluted by 87% in 15 minutes and by 97% in 30 minutes, and the memantine hydrochloride was eluted by 90% in 15 minutes and by 95% in 30 minutes.

That is, it was confirmed that the combined tables of Example 1 of the present invention improved the elution rate reduction phenomenon that may occur upon co-administration of Aricept® (Control 1) and Ebixa® (Control 1), which are commercially available, and both the donepezil hydrochloride and the memantine hydrochloride were eluted in an elution pattern similar to that of tables of Controls 1 and 2, which are commercial drugs.

In addition, FIG. 2A is a graph showing measurement results of the elution rate of donepezil hydrochloride of combined tablets prepared according to Examples 1 to 4 over time, and FIG. 2B is a graph showing measurement results of the elution rate of memantine hydrochloride of the combined tablets of Examples 1 to 4 over time.

Table 2 numerically shows the elution rate of donepezil hydrochloride, and Table 3 numerically shows the elution rate of memantine hydrochloride.

[Table 2]

| Classification | 0 min | 5 min | 10 min | 15 min | 30 min |
|---|---|---|---|---|---|
| Example 1 | 0 | 24.7 | 62.0 | 87.5 | 97.2 |
| Example 2 | 0 | 10.2 | 49.6 | 71.3 | 95.2 |

(continued)

| Classification | 0 min | 5 min | 10 min | 15 min | 30 min |
|---|---|---|---|---|---|
| Example 3 | 0 | 38.4 | 70.35 | 91.4 | 98.21 |
| Example 4 | 0 | 47.2 | 77.1 | 93.4 | 99.3 |

[Table 3]

| Classification | 0 min | 5 min | 10 min | 15 min | 30 min |
|---|---|---|---|---|---|
| Example 1 | 0 | 68.5 | 89.3 | 97.3 | 98.7 |
| Example 2 | 0 | 52.6 | 85.4 | 93.4 | 98.1 |
| Example 3 | 0 | 73.0 | 91.7 | 98.2 | 99.8 |
| Example 4 | 0 | 76.4 | 91.4 | 98.1 | 99.4 |

As illustrated in FIGS. 2A and 2B and Tables 2 and 3, upon co-elution of the combined tablets of Example 1 including donepezil hydrochloride and memantine hydrochloride, which were homogenously mixed without separation, donepezil was eluted by less than 35% for 5 minutes and by 80% or more during 15 minutes, and memantine was eluted by 60% or more for 5 minutes and by 90% or more for 15 minutes.

In contrast, in the combined preparation of Example 2, donepezil was eluted by less than 35% for 5 minutes and by 71% for 15 minutes, from which it was confirmed that a decrease in elution rate was exhibited after 15 minutes, and memantine was eluted by 60% or less for 5 minutes and by 90% or more for 15 minutes, from which it was confirmed that the initial elution rate was low.

In addition, in the cases of Examples 3 and 4, donepezil was eluted by 35% or more for 5 minutes and by 90% or more for 15 minutes, from which it was confirmed that the initial elution rate was high, and it was confirmed that memantine was eluted by 60% or more for 5 minutes and by 90% or more for 15 minutes.

Therefore, unlike the combined preparation of Example 1, the combined preparations of Examples 2 to 4 were not prepared in such a way that the elution of donepezil hydrochloride was suppressed to less than 35% for the initial 5 minutes similar to Control 1, and memantine hydrochloride was eluted by 60% or more for the initial 5 minutes similar to Control 2.

In addition, FIG. 3A is a graph showing the particle size of donepezil granules of Example 1, FIG. 3B is a graph showing the particle size of donepezil granules of Example 2, FIG. 3C is a graph showing the particle size of donepezil granules of Example 3, and FIG. 3D is a graph showing the particle size of donepezil granules of Example 4.

As illustrated in FIGS. 3A to 3D, it was confirmed that, as in Example 1, when the amount of particles having a size of 20 mesh or more to less than 50 mesh was between 15 wt% and 25 wt%, the amount of particles having a size of 50 mesh or more to less than 80 mesh is between 25 wt% to 35 wt%, the amount of particles having a size of 80 mesh or more to less than 200 mesh was between 20 wt% and 30 wt%, the amount of particles having a size of 200 mesh or more was between 15% and 25 wt%, and the amount of the particles having a size of 20 mesh or more to less than 50 mesh was smaller than that of the particles having a size of 200 mesh or more; particularly, when the particles having a size of 20 mesh were maintained in an amount of 5 wt% to 15 wt%, donepezil was eluted by less than 35% for 5 minutes and by 80% or more for 15 minutes.

In addition, FIG. 4A is a graph showing measurement results of the elution rate of donepezil hydrochloride of Control 1 and tablets prepared according to Comparative Examples 2 and 3 over time, and FIG. 4B is a graph showing measurement results of the elution rate of memantine hydrochloride of Control 1 and the tablets of Comparative Examples 2 and 3 over time.

Table 4 numerically shows the elution rate of donepezil hydrochloride, and Table 5 numerically shows the elution rate of memantine hydrochloride.

[Table 4]

| Classification | 0 min | 5 min | 10 min | 15 min | 30 min |
|---|---|---|---|---|---|
| Control 1 | 0 | 26.5 | 63.8 | 84.3 | 94.1 |
| Comparative Example 2 | 0 | 58.0 | 84.7 | 94.1 | 97.5 |
| Comparative Example 3 | 0 | 30.4 | 67.9 | 88.1 | 97.9 |

[Table 5]

| Classification | 0 min | 5 min | 10 min | 15 min | 30 min |
|---|---|---|---|---|---|
| Control 1 | 0 | 78.8 | 92.2 | 96.1 | 98.9 |
| Comparative Example 2 | 0 | 60.1 | 86.2 | 93.4 | 98.1 |
| Comparative Example 3 | 0 | 75.6 | 90.4 | 97.3 | 99.4 |

As illustrated in FIGS. 4A and 4B and Tables 4 and 5, upon elution of the tablets of Comparative Example 2, donepezil was eluted by less than 35% for 5 minutes and by 80% or more for 15 minutes, and memantine was eluted by 60% or more for 5 minutes and by 90% or more for 15 minutes, which is suitable for elution criteria, but the amount of related substance of the memantine hydrochloride reached an allowable reference level around week 4 in an accelerated test, from which it is confirmed that this is not a suitable preparation method.

**Experimental Example 2: Content Measurement of Related Substances**

The combined tablets of the examples and the comparative examples were subjected to an accelerated stability test after aluminum (Alu-Alu) packaging under 40% and 75% relative humidity, and a total content of related substances (%) over time was analyzed by gas chromatography every two weeks.

[Table 6]

| <Analysis conditions> | | | | |
|---|---|---|---|---|
| Detector | Hydrogen flame ionization detector | | | |
| Column | HP-ULTRA 2(50 m X 0.32 mm X 0.52 um) | | | |
| Flow rate | 4.0 ± 0.2 ml/min | | | |
| Carrier gas | Helium | | | |
| Column temperature | 85 ℃ | | | |
| Inlet temperature | 220 ℃ | | | |
| Injection amount | 3 ul | | | |
| Detector temperature | 300 ℃ | | | |
| Injection amount | 50 ul | | | |
| Column temperature conditions | Initial Temperature (℃) | Temperature Ramp (℃/min) | Final Temperature (℃) | Hold Time at Temperature(min) |
| | 50 | 0 | 50 | 2 |
| | 50 | 5 | 145 | 0 |
| | 145 | 10 | 250 | 20 |

[Table 7]

| Classification | Initial | | | 2 weeks | | | 4 weeks | | |
|---|---|---|---|---|---|---|---|---|---|
| | Related Substance E | Unidentified related substance | Total related substances | Related Substance E | Unidentified related substance | Total related substances | Related Substance E | Unidentified related substance | Total related substances |
| Control 1 | Non-detected | Non-detected | 0.0 | Non-detected | Non-detected | 0.0 | Non-detected | Non-detected | 0.0 |
| Control 2 | Non-detected | 0.06 | 0.06 | Non-detected | 0.07 | 0.07 | Non-detected | 0.06 | 0.06 |
| Example 1 | Non-detected | 0.05 | 0.05 | Non-detected | 0.08 | 0.08 | Non-detected | 0.07 | 0.07 |
| Comparative Example 2 | 0.02 | 0.05 | 0.07 | 0.03 | 0.12 | 0.15 | 0.03 | 0.19 | 0.22 |
| Comparative Example 3 | 0.03 | 0.04 | 0.07 | 0.04 | 0.11 | 0.15 | 0.04 | 0.18 | 0.22 |

As shown in Table 7, it was confirmed that the combined tablets of Example 1 exhibited a related substance content similar to that of Controls 1 and 2, which are commercial drugs.

When considering the related substance acceptance criteria of memantine tablets in accordance with the United States Pharmacopeia, which are set at 0.15% of related substance E (ethyl 4-acetamido-2-ethoxybenzoate), 0.20% of an unidentified related substance, and 0.5% of total related substances, it was confirmed that the amounts of the unidentified related substances of Comparative Examples 2 and 3 reached the acceptance criteria around week 4.

That is, it was confirmed that the cases of Comparative Examples 2 and 3, in which memantine hydrochloride was subjected to a wet process, were not stable to related substances as compared with Example 1.

**Experimental Example 3: Content Uniformity Measurement**

Content uniformity measurement was performed using the same method as the analysis method of the elution test of Experimental Example 1, and the test was conducted in accordance with the Formulation Uniformity Test Method in General Test Methods of the Korean Pharmacopoeia. The formulation uniformity test method is a test to show the uniformity of the amount of a main ingredient in each preparation.

For 10 samples, the content of a main ingredient in each preparation is measured using an appropriate method to calculate a determination value (when the cut-off value is 15 or less, it is determined that content uniformity is secured).

The determination value is calculated according to Equation 1 below of the formulation uniformity test method among general test methods of the Korean Pharmacopoeia.

[Equation 1]

$$\text{Determination value} = M - \text{mean content} + (2.3 \times \text{standard deviation})$$

The M value is as follows: 1) when a mean content is 98.5% to 101.5%, M = mean content;

2) when the mean content is less than 98.5%, M=98.5%; and

3) when the mean content is greater than 101.5%, M=101.5%.

Table 8 shows the content uniformity of donepezil, and Table 9 shows the content uniformity of memantine.

[Table 8]

| Classification | Mean (%, n=10) | Minimum (%) | Maximum (%) | Standard deviation | Determination value |
|---|---|---|---|---|---|
| Example 1 | 98.46 | 92.93 | 101.36 | 2.16 | 5.22 |
| Comparative Example 1 | 97.5 | 87.2 | 109.8 | 9.27 | 23.24 |
| Comparative Example 4 | 101.3 | 91.41 | 107.68 | 6.54 | 15.69 |

[Table 9]

| Classification | Mean (%, n=10) | Minimum (%) | Maximum (%) | Standard deviation | Determination value |
|---|---|---|---|---|---|
| Example 1 | 100.57 | 96.73 | 105.85 | 2.57 | 6.16 |
| Comparative Example 1 | 96.7 | 85.2 | 111.9 | 10.29 | 26.49 |
| Comparative Example 4 | 100.53 | 92.43 | 108.87 | 7.19 | 17.25 |

As shown in Tables 4 and 5, the case of Example 1 using an excipient having a particle size distribution similar to that of donepezil hydrochloride and memantine hydrochloride exhibited a small standard deviation and a suitable determination value (determination value=15 or less) as compared to the cases of Comparative Examples 1 and 4 using an excipient having a particle size distribution not similar to that of donepezil hydrochloride granules and memantine hydrochloride.

It was confirmed that, when an excipient having a particle size distribution similar to that of donepezil hydrochloride and memantine hydrochloride was used, stable content uniformity was secured.

**Experimental Example 4: Content Uniformity Measurement according to Particle Size of Excipient**

FIG. 5A is a graph showing the particle size of SD lactose, FIG. 5B is a graph showing the particle size of lactose hydrate, FIG. 5C is a graph showing the particle size of microcrystalline cellulose, Vivapur 12, and FIG. 5D is a graph showing the particle size of microcrystalline cellulose, MCC 102.

In addition, FIG. 6 is a graph showing the sizes of the classified granules of the combined tablets prepared according to Example 1 using lactose and microcrystalline cellulose having the above particle sizes.

As illustrated in FIGS. 5A to 5D, it was confirmed that both lactose and microcrystalline cellulose contained 50 wt% or more of particles having a size of 80 mesh upon sieving, and thus when tablets were prepared by adjusting a ratio thereof, content uniformity could be secured.

**INDUSTRIAL APPLICABILITY**

A pharmaceutical composition for preventing or treating dementia and cognitive dysfunction of the present invention can be prepared by mixing donepezil or a pharmaceutically acceptable salt thereof which is granulated by a wet process with memantine or a pharmaceutically acceptable salt thereof and directly compressing the resulting mixture, thereby addressing problems such as reduced efficacy due to a decrease in elution of a donepezil active ingredient upon co-administration of Aricept® (donepezil hydrochloride) and Ebixa® (memantine hydrochloride), which are commercially available, and enhancing a therapeutic effect through co-elution of the two materials in an immediate release manner within a short period of time.

In addition, the pharmaceutical composition of the present invention secures content uniformity by selecting an excipient having a particle size similar to that of particles of the active ingredients, exhibits enhanced stability by suppressing the generation of related substances, and further enhances medication compliance.

**Claims**

**1.** An immediate-release type pharmaceutical composition for preventing or treating dementia and cognitive dysfunction, which comprises memantine or a pharmaceutically acceptable salt thereof; and donepezil or a pharmaceutically acceptable salt thereof which is granulated by a wet process and

is directly compressed in a form of tablets.

2. The immediate-release type pharmaceutical composition of claim 1, wherein the granulated donepezil or a pharmaceutically acceptable salt thereof is included in an amount of 1 wt% to 20 wt%.

3. The immediate-release type pharmaceutical composition of claim 1, wherein the granulated donepezil or a pharmaceutically acceptable salt thereof and the memantine or a pharmaceutically acceptable salt thereof are mixed in a weight ratio of 1: 0.8 to 5.

4. The immediate-release type pharmaceutical composition of claim 1, wherein the granulated donepezil or a pharmaceutically acceptable salt thereof has a particle size distribution d(0.5) of 120 $\mu$m to 200 $\mu$m.

5. The immediate-release type pharmaceutical composition of claim 1, wherein the granulated donepezil or a pharmaceutically acceptable salt thereof comprises 15 wt% to 25 wt% of particles having a size of 20 mesh or more to less than 50 mesh, 25 wt% to 35 wt% of particles having a size of 50 mesh or more to less than 80 mesh, 20 wt% to 30 wt% of particles having a size of 80 mesh or more to less than 200 mesh, and 15 wt% to 25 wt% of particles having a size of at least 200 mesh, and
an amount of the particles having a size of 20 mesh or more to less than 50 mesh is smaller than that of the particles having a size of at least 200 mesh.

6. The immediate-release type pharmaceutical composition of claim 1, wherein particles having a size of 20 mesh of the granulated donepezil or a pharmaceutically acceptable salt thereof are included in an amount of 5 wt% to 15 wt%.

7. The immediate-release type pharmaceutical composition of claim 1, further comprising an excipient.

8. The immediate-release type pharmaceutical composition of claim 7, wherein the excipient is a mixture of lactose and microcrystalline cellulose in a weight ratio of 3 to 20: 1.

9. The immediate-release type pharmaceutical composition of claim 1, wherein the excipient comprises particles having a size of 80 mesh or more in an amount of 50 wt% or more.

10. A method of preparing an immediate-release type pharmaceutical composition for preventing or treating dementia and cognitive dysfunction, the method comprising:

   granulating donepezil or a pharmaceutically acceptable salt thereof by a wet process; and
   adding memantine or a pharmaceutically acceptable salt thereof to the granulated donepezil or a pharmaceutically acceptable salt thereof and directly compressing and homogenizing the resulting mixture.

**FIG. 1A**

Elution rate of donepezil hydrochloride (pH 1.2)

**FIG. 1B**

Elution rate of Memantine hydrochloride (pH 1.2)

FIG. 2A

Elution rate of donepezil hydrochloride (pH 1.2)

FIG. 2B

Elution rate of memantine hydrochloride (pH 1.2)

FIG. 3A

Donepezil granule 20 mesh

FIG. 3B

Donepezil granule 16 mesh

## FIG. 3C

Donepezil granule 25 mesh

## FIG. 3D

Donepezil granule 30 mesh

## FIG. 4A

## FIG. 4B

FIG. 5A

FIG. 5B

**FIG. 5C**

Vivapur 12

**FIG. 5D**

MCC 102

FIG. 6

## INTERNATIONAL SEARCH REPORT

| | International application No. |
|---|---|
| | **PCT/KR2017/010953** |

| A. CLASSIFICATION OF SUBJECT MATTER |
|---|
| *A61K 31/445(2006.01)i, A61K 31/13(2006.01)i* |
| According to International Patent Classification (IPC) or to both national classification and IPC |

| B. FIELDS SEARCHED |
|---|
| Minimum documentation searched (classification system followed by classification symbols) |
| A61K 31/445; A61K 45/06; A61K 31/13; A61P 25/28 |
| Documentation searched other than minimum documentation to the extent that such documents are included in the fields searched |
| Korean Utility models and applications for Utility models: IPC as above |
| Japanese Utility models and applications for Utility models: IPC as above |
| Electronic data base consulted during the international search (name of data base and, where practicable, search terms used) |
| eKOMPASS (KIPO internal) & Keywords: memantine, donepezil, dementia, cognitive skill, wet-type process, immediate release type, compression, tablet |

| C. DOCUMENTS CONSIDERED TO BE RELEVANT |||
|---|---|---|
| Category* | Citation of document, with indication, where appropriate, of the relevant passages | Relevant to claim No. |
| X | KR 10-1213345 B1 (EISAI R&D MANAGEMENT CO., LTD.) 17 December 2012 See claim 1; paragraphs [0003], [0049], [0059], [0083], [0087], [0096]; and figure 1. | 1-10 |
| X | US 2011-0251239 A1 (MOLINE, M. L. et al.) 13 October 2011 See abstract; claims 1-3, 5, 10, 13-23; and paragraphs [0104], [0106]-[0108]. | 1-10 |
| X | US 2006-0160852 A1 (KIMURA, S. et al.) 20 July 2006 See abstract; claims 1, 3, 6-8, 13-17; and paragraphs [0026], [0030], [0036], [0046]. | 1-10 |
| A | KR 10-1406456 B1 (ADAMAS PHARMACEUTICALS, INC.) 20 June 2014 See abstract; and claims 1-11. | 1-10 |
| A | KR 10-2005-0024296 A (H. LUNDBECK A/S.) 10 March 2005 See claims 1-15. | 1-10 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 05 JANUARY 2018 (05.01.2018) | **08 JANUARY 2018 (08.01.2018)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

INTERNATIONAL SEARCH REPORT

Information on patent family members

International application No.

**PCT/KR2017/010953**

| Patent document cited in search report | Publication date | Patent family member | Publication date |
|---|---|---|---|
| KR 10-1213345 B1 | 17/12/2012 | AU 2006-241771 A1 | 09/11/2006 |
| | | AU 2006-241771 B2 | 09/09/2010 |
| | | BR PI0608780 A2 | 09/11/2010 |
| | | CA 2604617 A1 | 09/11/2006 |
| | | CA 2604617 C | 17/06/2014 |
| | | CN 101166543 A | 23/04/2008 |
| | | CN 101166543 B | 16/07/2014 |
| | | EP 1878444 A1 | 16/01/2008 |
| | | EP 1878444 B1 | 09/09/2015 |
| | | IL 186179 A | 20/01/2008 |
| | | JP 5597343 B2 | 01/10/2014 |
| | | MX 2007013493 A | 21/01/2008 |
| | | NO 20075453 A | 28/01/2008 |
| | | NZ 562120 A | 30/07/2010 |
| | | RU 2007139712 A | 10/05/2009 |
| | | US 2009-0023778 A1 | 22/01/2009 |
| | | US 2014-0099366 A1 | 10/04/2014 |
| | | WO 2006-118265 A1 | 09/11/2006 |
| | | ZA 200708580 B | 29/10/2008 |
| US 2011-0251239 A1 | 13/10/2011 | WO 2011-127235 A1 | 13/10/2011 |
| US 2006-0160852 A1 | 20/07/2006 | AU 2005-320547 A1 | 06/07/2006 |
| | | AU 2005-320547 B2 | 05/02/2009 |
| | | AU 2005-320609 A1 | 06/07/2006 |
| | | AU 2005-320609 B2 | 02/07/2009 |
| | | BR PI0518396 A2 | 18/11/2008 |
| | | BR PI0519407 A2 | 20/01/2009 |
| | | CA 2592102 A1 | 06/07/2006 |
| | | CA 2592102 C | 24/01/2012 |
| | | CA 2592605 A1 | 06/07/2006 |
| | | CA 2592605 C | 07/12/2010 |
| | | CN 101090737 A | 19/12/2007 |
| | | CN 101090737 B | 08/09/2010 |
| | | CN 101090738 A | 19/12/2007 |
| | | CN 103550190 A | 05/02/2014 |
| | | EP 1830886 A1 | 12/09/2007 |
| | | EP 1830886 B1 | 13/04/2016 |
| | | EP 1832298 A1 | 12/09/2007 |
| | | HK 1112399 A1 | 27/01/2017 |
| | | IL 183650 A | 31/12/2013 |
| | | IL 183871 A | 31/10/2007 |
| | | JP 2008-525313 A | 17/07/2008 |
| | | JP 2012-144568 A | 02/08/2012 |
| | | JP 4999466 B2 | 15/08/2012 |
| | | JP 5514249 B2 | 04/06/2014 |
| | | KR 10-0866720 B1 | 05/11/2008 |
| | | KR 10-0904602 B1 | 25/06/2009 |
| | | KR 10-2007-0086667 A | 27/08/2007 |

Form PCT/ISA/210 (patent family annex) (January 2015)

INTERNATIONAL SEARCH REPORT
Information on patent family members

International application No.

**PCT/KR2017/010953**

| Patent document cited in search report | Publication date | Patent family member | Publication date |
|---|---|---|---|
| | | KR 10-2007-0089207 A | 30/08/2007 |
| | | MX 2007007835 A | 20/08/2007 |
| | | MX 2007007836 A | 20/08/2007 |
| | | NO 20073461 A | 27/09/2007 |
| | | NZ 555693 A | 29/10/2010 |
| | | NZ 555901 A | 29/10/2010 |
| | | RU 2007128767 A | 10/02/2009 |
| | | RU 2007128768 A | 10/02/2009 |
| | | US 2006-0159753 A1 | 20/07/2006 |
| | | US 2006-0246003 A1 | 02/11/2006 |
| | | US 2006-0280789 A1 | 14/12/2006 |
| | | US 2007-0129402 A1 | 07/06/2007 |
| | | US 2008-0213368 A1 | 04/09/2008 |
| | | US 2009-0208579 A1 | 20/08/2009 |
| | | US 2010-0152164 A1 | 17/06/2010 |
| | | US 2011-0045074 A1 | 24/02/2011 |
| | | US 8481565 B2 | 09/07/2013 |
| | | US 8507527 B2 | 13/08/2013 |
| | | WO 2006-070781 A1 | 06/07/2006 |
| | | WO 2006-070930 A1 | 06/07/2006 |
| | | ZA 200705118 B | 25/09/2008 |
| | | ZA 200705119 B | 25/06/2008 |
| KR 10-1406456 B1 | 20/06/2014 | AT 481096 T | 15/10/2010 |
| | | AU 2005-209310 A1 | 11/08/2005 |
| | | AU 2005-209310 B2 | 06/01/2011 |
| | | AU 2005-215767 A1 | 01/09/2005 |
| | | AU 2005-215775 A1 | 01/09/2005 |
| | | AU 2005-215775 B2 | 03/02/2011 |
| | | AU 2005-309601 A1 | 01/06/2006 |
| | | AU 2006-244297 A1 | 16/11/2006 |
| | | BR PI0518483 A2 | 18/11/2008 |
| | | BR PI0607017 A2 | 04/08/2009 |
| | | CA 2554959 A1 | 11/08/2005 |
| | | CA 2556214 A1 | 01/09/2005 |
| | | CA 2556216 A1 | 01/09/2005 |
| | | CA 2588295 A1 | 01/06/2006 |
| | | CA 2588295 C | 22/10/2013 |
| | | CA 2604052 A1 | 16/11/2006 |
| | | CA 2604052 C | 08/07/2014 |
| | | CN 101060830 A | 24/10/2007 |
| | | CN 101247795 A | 20/08/2008 |
| | | CN 101247795 B | 01/02/2012 |
| | | CN 101686945 A | 31/03/2010 |
| | | CN 1929830 A | 14/03/2007 |
| | | DE 05852057 T1 | 29/11/2007 |
| | | DK 1874282 T3 | 25/10/2010 |
| | | EP 1715843 A1 | 02/11/2006 |
| | | EP 1727538 A2 | 06/12/2006 |
| | | EP 1734920 A2 | 27/12/2006 |

Form PCT/ISA/210 (patent family annex) (January 2015)

**INTERNATIONAL SEARCH REPORT**
Information on patent family members

International application No.

**PCT/KR2017/010953**

| Patent document cited in search report | Publication date | Patent family member | Publication date |
|---|---|---|---|
| | | EP 1789028 A2 | 30/05/2007 |
| | | EP 1827385 A2 | 05/09/2007 |
| | | EP 1827385 B1 | 27/03/2013 |
| | | EP 1874282 A2 | 09/01/2008 |
| | | EP 1874282 B1 | 15/09/2010 |
| | | EP 2243475 A1 | 27/10/2010 |
| | | EP 2243475 B1 | 13/01/2016 |
| | | EP 2343057 A1 | 13/07/2011 |
| | | ES 2351709 T3 | 09/02/2011 |
| | | HK 1103517 A1 | 22/11/2013 |
| | | HK 1106711 A1 | 08/07/2011 |
| | | IL 183384 A | 20/09/2007 |
| | | IL 186504 A | 20/01/2008 |
| | | JP 2007-522248 A | 09/08/2007 |
| | | JP 2007-522249 A | 09/08/2007 |
| | | JP 2008-520736 A | 19/06/2008 |
| | | JP 2008-535867 A | 04/09/2008 |
| | | JP 2009-173669 A | 06/08/2009 |
| | | JP 5666087 B2 | 12/02/2015 |
| | | KR 10-1301429 B1 | 30/08/2013 |
| | | KR 10-2006-0124731 A | 05/12/2006 |
| | | KR 10-2007-0017133 A | 08/02/2007 |
| | | KR 10-2007-0017136 A | 08/02/2007 |
| | | MX 2007006120 A | 07/12/2007 |
| | | MX 2007012374 A | 22/02/2008 |
| | | RU 2007122410 A | 27/12/2008 |
| | | RU 2007140348 A | 20/05/2009 |
| | | SG 157415 A1 | 29/12/2009 |
| | | US 2005-0209218 A1 | 22/09/2005 |
| | | US 2005-0245460 A1 | 03/11/2005 |
| | | US 2005-0245617 A1 | 03/11/2005 |
| | | US 2006-0052370 A1 | 09/03/2006 |
| | | US 2006-0142398 A1 | 29/06/2006 |
| | | US 2006-0252788 A1 | 09/11/2006 |
| | | US 2009-0253728 A1 | 08/10/2009 |
| | | US 2009-0306051 A1 | 10/12/2009 |
| | | US 2010-0022659 A1 | 28/01/2010 |
| | | US 2010-0047342 A1 | 25/02/2010 |
| | | US 2010-0260838 A1 | 14/10/2010 |
| | | US 2010-0266684 A1 | 21/10/2010 |
| | | US 2010-0292216 A1 | 18/11/2010 |
| | | US 2010-0311697 A1 | 09/12/2010 |
| | | US 2011-0059169 A1 | 10/03/2011 |
| | | US 2011-0064804 A1 | 17/03/2011 |
| | | US 2012-0046365 A1 | 23/02/2012 |
| | | US 2012-0264782 A1 | 18/10/2012 |
| | | US 2012-0264783 A1 | 18/10/2012 |
| | | US 2012-0264829 A1 | 18/10/2012 |
| | | US 2012-0264978 A1 | 18/10/2012 |
| | | US 2012-0288560 A1 | 15/11/2012 |

Form PCT/ISA/210 (patent family annex) (January 2015)

INTERNATIONAL SEARCH REPORT
Information on patent family members

International application No.

PCT/KR2017/010953

| Patent document cited in search report | Publication date | Patent family member | Publication date |
|---|---|---|---|
| | | US 2013-0131110 A1 | 23/05/2013 |
| | | US 2013-0165527 A1 | 27/06/2013 |
| | | US 2014-0134243 A1 | 15/05/2014 |
| | | US 2014-0135529 A1 | 15/05/2014 |
| | | US 2014-0179797 A1 | 26/06/2014 |
| | | US 2014-0336266 A1 | 13/11/2014 |
| | | US 2015-0297537 A1 | 22/10/2015 |
| | | US 2016-0220545 A1 | 04/08/2016 |
| | | US 2016-0243035 A1 | 25/08/2016 |
| | | US 2016-0243058 A1 | 25/08/2016 |
| | | US 2016-0243093 A1 | 25/08/2016 |
| | | US 2016-0243094 A1 | 25/08/2016 |
| | | US 2016-0243095 A1 | 25/08/2016 |
| | | US 2016-0250149 A1 | 01/09/2016 |
| | | US 2016-0250161 A1 | 01/09/2016 |
| | | US 2017-0056340 A1 | 02/03/2017 |
| | | US 7619007 B2 | 17/11/2009 |
| | | US 8058291 B2 | 15/11/2011 |
| | | US 8168209 B2 | 01/05/2012 |
| | | US 8173708 B2 | 08/05/2012 |
| | | US 8283379 B2 | 09/10/2012 |
| | | US 8293794 B2 | 23/10/2012 |
| | | US 8329752 B2 | 11/12/2012 |
| | | US 8338485 B2 | 25/12/2012 |
| | | US 8338486 B2 | 25/12/2012 |
| | | US 8362085 B2 | 29/01/2013 |
| | | US 8426472 B2 | 23/04/2013 |
| | | US 8580858 B2 | 12/11/2013 |
| | | US 8598233 B2 | 03/12/2013 |
| | | WO 2005-072705 A1 | 11/08/2005 |
| | | WO 2005-079756 A2 | 01/09/2005 |
| | | WO 2005-079756 A3 | 22/09/2005 |
| | | WO 2005-079773 A2 | 01/09/2005 |
| | | WO 2005-079773 A3 | 27/10/2005 |
| | | WO 2006-024018 A2 | 02/03/2006 |
| | | WO 2006-024018 A3 | 20/07/2006 |
| | | WO 2006-058059 A2 | 01/06/2006 |
| | | WO 2006-058059 A3 | 06/07/2006 |
| | | WO 2006-121560 A2 | 16/11/2006 |
| | | ZA 200704233 B | 26/11/2008 |
| | | ZA 200709535 B | 26/11/2008 |
| KR 10-2005-0024296 A | 10/03/2005 | AR 040121 A1 | 16/03/2005 |
| | | AT 414519 T | 15/12/2008 |
| | | AU 2003-227516 A1 | 19/12/2003 |
| | | AU 2003-227516 B2 | 20/03/2008 |
| | | BR 0311375 A | 15/03/2005 |
| | | CA 2426492 A1 | 16/09/2003 |
| | | CA 2426492 C | 03/10/2006 |
| | | CA 2558708 A1 | 16/09/2003 |

Form PCT/ISA/210 (patent family annex) (January 2015)

INTERNATIONAL SEARCH REPORT
Information on patent family members

International application No.

**PCT/KR2017/010953**

| Patent document cited in search report | Publication date | Patent family member | Publication date |
|---|---|---|---|
| | | CN 1655793 A | 17/08/2005 |
| | | CY 1108725 T1 | 09/04/2014 |
| | | DK 1509232 T3 | 23/02/2009 |
| | | EA 007632 B1 | 29/12/2006 |
| | | EA 200401617 A1 | 30/06/2005 |
| | | EP 1509232 A1 | 02/03/2005 |
| | | EP 1509232 B1 | 19/11/2008 |
| | | ES 2314200 T3 | 16/03/2009 |
| | | IS 7558 A | 26/11/2004 |
| | | JP 2005-528431 A | 22/09/2005 |
| | | MX PA04011762 A | 31/03/2005 |
| | | NO 20045434 A | 13/12/2004 |
| | | NO 332754 B1 | 07/01/2013 |
| | | NZ 536603 A | 29/06/2007 |
| | | PE 06232004 A1 | 11/09/2004 |
| | | PL 373903 A1 | 19/09/2005 |
| | | WO 03-101458 A1 | 11/12/2003 |
| | | ZA 200409147 B | 28/06/2006 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1213345

- KR 20090016611

**Non-patent literature cited in the description**

- **PIERRE N. TARIOT et al.** memantine Treatment in Patients with Moderate to Severe Alzheimer Disease Already Receiving Donepezil a Randomized Controlled trial. *JAMA,* vol. 291 (3), 317-324